Europäisches Patentamt

European Patent Office

Office européen des brevets

(19)

(11) Veröffentlichungsnummer: **0 004 940**
**B1**

(12) **EUROPÄISCHE PATENTSCHRIFT**

(45) Veröffentlichungstag der Patentschrift: **30.09.81**

(21) Anmeldenummer: **79101113.3**

(22) Anmeldetag: **11.04.79**

(51) Int. Cl.³: **G 01 N 33/50,** **C 07 G 7/00,**
**G 21 H 5/00**

(54) **Verfahren zur radioimmunologischen Bestimmung von Prokollagen (Typ III) und Prokollagen-Peptid (Typ III), zur Herstellung von für das Verfahren geeignetem Prokollagen-Peptid (Typ III) und zur Herstellung von Anti-Prokollagen-Peptid (Typ III)-Serum.**

(30) Priorität: **18.04.78 DE 2816841**

(43) Veröffentlichungstag der Anmeldung:
**31.10.79 Patentblatt 79/22**

(45) Bekanntmachung des Hinweises auf die Patenterteilung:
**30.09.81 Patentblatt 81/39**

(84) Benannte Vertragsstaaten:
**BE CH DE FR GB IT LU NL SE**

(73) Patentinhaber: **Max-Planck-Gesellschaft zur**
**Förderung der Wissenschaften e.V.**
**Bunsenstrasse 10**
**D-3400 Göttingen (DE)**

(72) Erfinder: **Timpl, Rupert, Dr.**
**Bergstrasse 18**
**D-8033 Krailling (DE)**

(74) Vertreter: **Weickmann, Heinrich, Dipl.-Ing. et al,**
**Postfach 860 820 Möhlstrasse 22**
**D-8000 München 86 (DE)**

(56) Entgegenhaltungen:
**DE - A - 1 598 945**

**ANGEWANDTE CHEMIE, 88. Jahrgang, Nr. 17,**
**1976, Weinheim**
**H.G. ECKERT, "Die Technik des Radio-**
**immunoassays". Seiten 565 bis 573**

**INTERNATIONAL ARCHIVES OF ALLERGY, Band**
**29, 1966, Basel**
**P.J. McCONAHEY et al. "A Method of Trace**
**Iodination of Proteins for Immunoligic Studies".**
**Seiten 185 bis 189**

**MEDIZINAL-MARKT/ACTA MEDICOTECHNICA,**
**25. Jahrgang, Nr. 10, 1977, Berlin**
**H. MEINHOLD, "Nuklearmedizinische in-vitro-**
**Diagnostik im Submikrobereich". Seiten 317 bis**
**322**

Verfahren zur radioimmunologischen Bestimmung von Prokollagen (Typ III) und Prokollagen-Peptid (Typ III), zur Herstellung von für das Verfahren geeigneten Prokollagen-Peptid (Typ III) und zur Herstellung von Anti-Prokollagen-Peptid (Type III)-Serum

Die Erfindung betrifft ein Verfahren zur radioimmunologischen Bestimmung von Prokollagen (Typ III) und Prokollagen-Peptid (Typ III) und die Herstellung eines für dieses Verfahren geeigneten Prokollagen-Peptids (Typ III).

Prokollagen (Typ III) ist eine biosynthetische Vorläuferform eines speziellen Kollagens (Typ III), das hauptsächlich im retikulären Bindegewebe vorkommt. Es unterscheidet sich von Kollagen (Typ III) durch ein zusätzliches, am Aminoende lokalisiertes Peptidsegment (Prokollagen-Peptid (Typ III)), das sich durch Behandlung mit Kollagenase vom Molekül abspalten läßt.

Neuere Immunofluoreszenz-Untersuchungen haben gezeigt, daß fibrosierende Prozesse, die zB. bei Leberzirrhose und Hepatitis auftreten, von einem hohen Umsatz von Prokollagen (Typ III) und Prokollagen-Peptid (Typ III) begleitet sind. Der Nachweis dieser im Blut zirkulierenden Antigene erlaubt deshalb eine frühzeitige Erkennung derartiger Erkrankungen.

Immunhistologische Untersuchungen gestatten zwar einen spezifischen Nachweis dieser Antigene, lassen sich aber nicht quantitativ auswerten. Dadurch ist die Aussagekraft derartiger Methoden begrenzt.

Der Erfindung liegt daher die Aufgabe zugrunde, dieses Problem zu lösen und ein quantitatives, rasch und einfach durchzuführendes Verfahren zur Bestimmung dieser Antigene zu schaffen.

Es wurde nun gefunden, daß eine quantitative Messung derartiger Antigene durch eine radioimmunologische Bestimmungsmethode möglich ist. Radioimmunologische Bestimmungen sind zwar an sich bekannt, z. B. aus Angewandte Chemie *88*, 565—573 (1976), wo die Bestimmung von Hepatitis-virusantigen beschrieben wird. Es war jedoch nicht vorhersehbar, daß diese Methode für die Bestimmung der erfindungsgemäß zu bestimmenden Antigene brauchbar sein würde, da Bindegewebskomponenten, zu denen sie gehören, vielfach unlöslich abgelagert werden, sehr rasch abgebaut werden und daher die diagnostische Relevanz ihrer Konzentration im Serum überraschend ist.

Ein Gegenstand der Erfindung ist daher ein Verfahren zur radioimmunologischen Bestimmung von Prokollagen (Typ III) und Prokollagen-Peptid (Typ III), bei dem eine bestimmte Menge radioaktiv markiertes Prokollagen (Typ III) oder Prokollagen-Peptid (Typ III) und ein hochspezifisches Anti-Prokollagen-(Typ III)-Serum oder ein Anti-Prokollagen-Peptid-(Typ III)-Serum gemeinsam mit einer Probe mit unbekanntem Gehalt an Prokollagen (Typ III) oder Prokollagen-Peptid (Typ III) zur Reaktion gebracht werden. In der vom Radio-Immuno-Assay (RIA) an sich bekannten Weise konkurriert hierbei das radioaktive markierte Prokollagen bzw. Prokollagen-Peptid mit dem in der zu bestimmenden Probe enthaltenen, nichtmarkierten Prokollagen oder Prokollagen-Peptid um den Antikörper, so daß im gebildeten Antigen-Antikörper-Komplex die Radioaktivität um so geringer ist, je mehr nichtmarkiertes Prokollagen bzw. Prokollagen-Peptid in der zu bestimmenden Probe enthalten ist. Der gebildete unlösliche Antigen-Antikörper-Komplex kann in üblicher Weise aus der Lösung abgetrennt und die darin enthaltene Radioactivität bestimmt werden. Alternativ ist es auch möglich, die in der Lösung, also im Überstand der Abtrennung des Antigen-Antikörper-Komplexes, verbleibende Radioactivität zu messen. Anhand einer Eichkurve, die mittels Proben von bekanntem Gehalt an Prokollagen oder Prokollagen-Peptid erstellt wurde, läßt sich dann die in der zu untersuchenden Probe enthaltene Menge Prokollagen oder Prokollagen-Peptid feststellen.

Die Trennung des Antigen-Antikörper-Komplexes von der Lösung kann nach den hierfür dem Fachmann bekannten, üblichen Methoden erfolgen, wie Abfiltrieren, Absaugen, Abzentrifugieren und dergleichen. Auch ist es möglich, das Antiserum an einen festen Träger, beispielsweise die Innenwand eines Reagenzglases gebunden, anzuwenden.

Vorzugsweise wird das Verfahren so durchgeführt, daß die Trennung des mit dem hochspezifischen Anti-Prokollagen-(Typ III)-Serum oder mit dem Anti-Prokollagen-Peptid-(Typ III)-Serum gebildeten Antigen-Antikörper-Komplexes von dem nichtumgesetzten Antigen durch die Verwendung eines gegen das hochspezifische Serum gerichteten zweiten Antikörpers erfolgt. Bevorzugt wird hierfür ein Antikörper gegen $\gamma$-Imunoglobulin der für die Gewinnung des Antiserums verwendeten Tierart.

Die Markierung des Antigens, d. h. des Prokollagens (Typ III) oder Prokollagen-Peptids (Typ III) mit dem Radionuclid, kann mit den für die Radiomarkierung von Proteinen bekannten Methoden durchgeführt werden. Als Radionuclid wird vorzugsweise Jod 125 verwendet. Zur Markierung mit diesem Radionuclid wird die Chloramin-T-Methode (Int. Arch. Allergy, *29* 185) bevorzugt.

Für die erfindungsgemäße Bestimmungsmethode ist es entscheidend, daß eine geeignete Quelle zur Gewinnung von Prokollagen-Peptid (Typ III) zur Verfügung steht. Es hat sich nun gezeigt, daß die Herstellung von humanem oder tierischem, hochgereinigtem Prokollagen-Peptid (Typ III) aus Tiergewebe oder pathologischem Körperflüssigkeiten möglich ist, wenn das Gewebe mit Kollagenase abgebaut und das Prokollagen oder Prokollagen-Peptid aus dem Kollagenase-Extrakt oder der Körperflüssigkeit abgetrennt und durch Kombination von chromatographischen Methoden und/oder Immunadsorption gereinigt wird.

Ein weiterer Gegenstand der Erfindung ist daher ein Verfahren zur Herstellung eines für das erfindungsgemäße Bestimmungsverfahren geeigneten hochgereinigten Prokollagen-Peptids (Typ III), welches dadurch gekennzeichnet ist, daß humanes oder tierisches Gewebe oder pathologische Körperflüssigkeit oder Kollagen-Extrakte derselben mit Kollagenase abgebaut und das dabei gebildete Prokollagen oder Prokollagen-Peptid aus dem Kollagenase-Extrakt oder der Körperflüssigkeit abgetrennt und chromatographisch oder durch Immunadsorption oder durch Kombination dieser Methoden gereinigt wird.

Als Tiergewebe haben sich fetale Kälberhaut und als Körperflüssigkeit humane Ascites-Flüssigkeit besonders ergiebig zur Gewinnung des Prokollagen-Peptids (Typ III) erwiesen.

Die Reinigung mittels Immunadsorption wird zweckmäßig wie folgt durchgeführt: In einem ersten Schritt werden gereinigte Antikörper gegen Prokollagen-Peptid (Typ III) unlöslich gemacht. Die Reinigung der verwendeten Antikörper erfolgt zweckmäßig über Affinitätschromatographie, es können jedoch auch die anderen für die Antikörperreinigung an sich bekannten Methoden angewendet werden. Bevorzugt werden aus Kaninchen gewonnene Antikörper verwendet. Die Unlöslichmachung erfolgt durch Fixierung an einen festen Träger nach den bekannten Methoden der Fixierung biologisch aktiver Proteine an feste Träger. Bevorzugt erfolgt die Bindung an mit Bromcyan aktivierte Agarose oder diazotierte p-Aminobenzylcellulose. Mit dem so hergestellten Antikörperadsorbens werden die gegebenenfalls vorgereinigten Extrakte bzw. Körperflüssigkeiten dann inkubiert. Das Prokollagen-Peptid (Typ III) bindet hierbei an das Antikörperadsorbens und wird dann, zweckmäßigerweise nach Waschen des Trägers, mit geeigneten Elutionsmitteln wieder eluiert. Geeignete Elutionsmittel lassen sich durch einfache Vorversuche leicht ermitteln. Besonders geeignet erwies sich etwa 3 molare KSCN-Lösung für die Elution, jedoch lassen sich selbstverständlich auch andere Salzlösungen verwenden.

Das so hergestellte gereinigte Prokollagen-Peptid (Typ III) wird dann zur Immunisierung verwendet und so nach den üblichen Methoden der Antiserumgewinnung ein hochspezifisches Anti-Prokollagen-Peptid-(Typ III)-Serum hergestellt. Zweckmäßigerweise erfolgt die Immunisierung durch subkutane Injektion von Prokollagen-Peptid (Typ III) in Versuchstiere, vorzugsweise Kaninchen, in Gegenwart des kompletten Freund'schen Adjuvans. In diesem bevorzugten Fall beträgt die Antigendosis dabei zweckmäßigerweise etwa 2 mg/Tier.

Der erfindungsgemäße Radio-Immun-Test erlaubt die Messung von Konzentrationen bis in den Bereich von 1 ng/ml. Damit ist es möglich, den Test zur Feststellung von Antigen in Humanseren einzusetzen. Ein Vergleich von Probanden mit Leberfibrose zeigte einen bis zu zehnfachen Anstieg in der Konzentration des Antigens, wodurch Lebererkrankungen sicher festgestellt werden können.

Die folgenden Beispiele erläutern die Erfindung weiter.

Beispiel 1
Durchführung des Radio-Immun-Tests:

25 $\mu$g Prokollagen-Peptid (Typ III) werden mit 1 MilliCurie Jod 125 nach der Chloramin-T-Methode markiert und ungebundenes Jod durch Dialyse entfernt. Die weiteren Schritte bei der Herstellung des Radio-Immun-Tests werden vorzugsweise in Gegenwart von 0,04 % eines nichtionischen Detergens, wie z. B. Tween 20, Durchgeführt. Bindungskurven mit Antikörper werden mit 2 ng markiertem Peptid bestimmt. Die Konzentration an Prokollagen-Peptid (Typ III) in einer unbekannten Proke von Serum oder anderen Körperflüssigkeiten wird in folgendem Inhibitionstest bestimmt:

Eine bestimmte Menge des Antikörpers wird mit der unbekannten Probe 6 Stunden bei 4°C vorinkubiert und nach Zugabe von 2 ng markiertem Peptid weitere 12 Stunden bei 4°C inkubiert. Danach wird ein Überschuß von Antikörper gegen Kaninchen-$\gamma$-Immunglobulin zugesetzt und das im Immunkomplex gebundene Antigen aus der Lösung abgetrennt. Die Inhibitionsaktivität der unbekannten Probe wird mit der Aktivität einer Standard-Konzentration von nichtmarkiertem Prokollagen-Peptid (Typ III) verglichen.

Beispiel 2
Herstellung von Prokollagen-Peptid (Typ III):

Prokollagen-Peptid (Typ III) wird hergestellt durch Einwirkung von Kollagenase auf Prokollagen (Typ III) bei 37°C. Hierbei wird das Peptid keinen Denaturierungsmitteln ausgesetzt. Zur Herstellung größerer Mengen des Peptids wird ein modifiziertes Verfahren angewendet. Alle Verfahrensschritte bis zur Einwirkung der Kollagenase werden im Kälteraum durchgeführt. Die verschiedenen NaCl-Lösungen, die zur Löslichmachung eingesetzt werden, enthalten 0,05 M Tris-HCL, pH 7,4 0·01 M ÄDTA, Natriumazid (200 mg/ml) und die Proteasen-Inhibitoren Phenylmethylsulfonyl-fluorid (3 $\mu$g/ml) und p-Chlormercuribenzoat (3 $\mu$g/ml).

Fetale Kälberhaut (3 kg) wird in 10 l 1 M NaCL homogenisiert und zwei Tage Lang extrahiert Gelöstes Kollagen wird aus dem Extrakt gefällt durch Zugabe von festem NaCl bis zu einer Endkonzentration von 2,5 M. Nach Rühren über Nacht wird das Präzipitat durch Zentrifugierung gesammelt (1800 xg, 20 Minuten), zweimal mit 2,5 M NaCl gewaschen und wieder aufgelöst, indem es über Nacht in 10 l 0,5 M NaCl gerührt wird. Kleine Mengen unlöslichen Materials werden durch Zentrifugierung entfernt. Die so erhaltene Mischung von Kollagen (Typ III) und Prokollagen (Typ III) wird dann mit 1,6 M NaCl gefällt. Das Präzipitat wird dann in 2 l 0,05 M Tris-HCL (pH 8,0) suspendiert und nach Zusatz von 0,02 M CaCl$_2$ 20 Minuten lang bei 50°C erhitzt und anschließend 3 Stunden bei 37°C zusammen mit 1500 Einheiten bakterieller Kollagenase pro Gramm feuchtes Präzipitat inkubiert. Nach Einwirkung der Kollagenase wird das gebildete Präzipitat durch Zentrifugierung abgetrennt und die Lösung dialysiert gegen 0,005 M Tris-HCl, pH 8,6, 8 M Harnstoff und über die DEAE-Cellulosesäule (5,0 × 30 cm) gegeben, die mit dem gleichen Puffer äquilibriert wurde.

Die auf der Säule gebundenen Proteine werden mit NaCl-Lösungen ausgewaschen, deren Konzentration von 0 bis 0,3 M ansteigt. Die gesamte Elutionsmenge beträgt 2 l. Die aus der Säule ausfließende Lösung wird bezüglich der Adsorption bei 236 nm und ihrer Antigenaktivität durch Verwendung von Antikörpern überprüft, die spezifisch für das amino-terminale Segment des Prokollagens (Typ III) sind. Normalerweise enthält der letzte Peak, der aus der Säule eluiert wird, das Prokollagen-Peptid (Typ III). Das Peptid wird durch Dialyse gegen destilliertes Wasser entsalzt und lyophilisiert. Die weitere Reinigung erfolgt auf einer Säule mit Agarose A 1,5 M (2 × 120 cm), die mit 1 M CaCl$_2$, 0,05 M Tris-HCL, pH 7,5, äquilibriert ist.

**Patentansprüche**

1. Verfahren zur radioimmunologischen Bestimmung von Prokollagen (Typ III) und Prokollagen-Peptid (Typ III), dadurch gekennzeichnet, daß eine bestimmte Menge radioaktiv markiertes Prokollagen (Typ III) oder Prokollagen-Peptid (Typ III) und ein hochspezifisches Anti-Prokollagen-(Typ III)-Serum bzw. Anti-Prokollagen-Peptid-(Typ III)-Serum gemeinsam mit einer Probe mit unbekanntem Gehalt an Prokollagen (Typ III) oder Prokollagen-Peptid (Typ III) zur Reaktion gebracht werden, der gebildete Antigen-Antikörper-Komplex abgetrennt und die Radioaktivität des Komplexes oder des Überstandes gemessen wird.

2. Verfahren nach Anspruch 1, dadurch gekennzeichnet, daß ein mit einem Radionuclid markiertes Prokollagen (Typ III) oder Prokollagen-Peptid (Typ III) verwendet wird.

3. Verfahren nach Anspruch 2, dadurch gekennzeichnet, daß als Radionuclid Jod 125 verwendet wird.

4. Verfahren nach einem der vorhergehenden Ansprüche, dadurch gekennzeichnet, daß der aus Anti-Prokollagen-(Typ III)-Serum bzw. Anti-Prokollagen-Peptid-(Typ III)-Serum und Prokollagen (Typ III) bzw. Prokollagen-Pepti (Typ III) gebildete Antigen-Antikörper-Komplex von nichtumgesetztem Antigen durch Zusatz eines gegen das hochspezifische Antiserum gerichteten zweiten Antikörpers und Abtrennung des damit gebildeten Komplexes von Überstand, abgetrennt wird.

5. Verfahren zur Herstellung von für das Verfahren von Anspruch 1 bis 4 geeignetem hochgereinigtem Prokollagen-Peptid (Typ III), dadurch gekennzeichnet, daß humanes oder tierisches Gewebe oder pathologische Körperflüssigkeiten bzw. Kollagenextrakte davon mit Kollagenase abgebaut und das dabei gebildete Prokollagen oder Prokollagen-Peptid abgetrennt und durch Kombination von chromatographischen Methoden und/oder Immunadsorption gereinigt wird.

6. Verfahren nach Anspruch 5, dadurch gekennzeichnet daß als Tiergewebe fetale Kälberhaut bzw. als Körperflüssigkeit humane Ascitesflüssigkeit eingesetzt wird.

7. Verfahren zur Herstellung eines hochspezifischen Anti-Prokollagen-Peptid-(Typ III)-Serums, dadurch gekennzeichnet, daß ein nach Anspruch 5 oder 6 hergestelltes Prokollagen-Peptid (Typ III) zur Immunisierung von Versuchstieren verwendet und deren Serum gewonnen wird.

**Claims**

1. Process for the radioimmunological determination of procollagen (type III) and procollagen peptide (type III), characterised in that a definite amount of radio-actively marked procollagen (type III) or procollagen peptide (type III) and a highly specific anti-procollagen (type III) serum or anti-procollagen peptide (type III) serum, together with a sample with an unknown content of procollagen (type III) or of procollagen peptide (type III), are brought to reaction, the antigen-antibody complex formed is separated off and the radioactivity of the complex or of the supernatant is measured.

2. Process according to claim 1, characterised in that a procollagen (type III) or procollagen peptide (type III) marked with a radionuclide is used.

3. Process according to claim 2, characterised in that iodine 125 is used as radionuclide.

4. Process according to one of the preceding claims, characterised in that the antigen-antibody complex formed from anti-procollagen (type III) serum or anti-procollagen peptide (type III) serum and procollagen (type III) or procollagen peptide (type III) is separated from unreacted antigen by the addition of a second antibody directed against the highly specific antiserum and separation of the thus formed complex from the supernatant.

5. Process for the preparation of highly purified procollagen peptide (type III) suitable for the process of claim 1 to 4, characterised in that human and animal tissue or pathological body fluids or collagen extracts thereof is broken down with collagenase and the procollagen or procollagen peptide thereby formed is separated off and purified by a combination of chromatographic methods and/or immune adsorption.

6. Process according to claim 5, characterised in that as animal tissue there is used foetal calf skin or as body fluid human ascitic fluid.

7. Process for the preparation of a highly specific anti-procollagen peptide (type III) serum, characterised in that a procollagen peptide (type III) prepared according to claim 5 or 6 is used for the immunisation of experimental animals and their serum is obtained.

**Revendications**

1. Procédé pour le dosage radio-immunologique du procollagène (type III) et du peptide de procollagène (type III), caractérisé en ce qu'une quantité déterminée de procollagène (type III) ou de peptide de procollagène (type III) marqué par voie radio-active et un sérum anti-procollagène (type III) ou un sérum anti-peptide de procollagène (type III) hautement spécifique sont amenés, conjointement avec un échantillon à teneur inconnue en procollagène (type III) ou en peptide de procollagène (type III), à réagir, le complexe antigène-anticorps formé est séparé et la radio-activité du complexe ou du résidu est mesurée.

2. Procédé suivant la revendication 1, caractérisé en ce qu'un procollagène (type III) ou un peptide de procollagène (type III) marqué par un radionucléide est utilisé.

3. Procédé suivant la revendication 2, caractérisé en ce que de l'iode 125 est utilisée en tant que radionucléide.

4. Procédé suivant l'une des revendications précédentes, caractérisé en ce que le complexe antigène-anticorps formé à partir de sérum anti-procollagène (type III) ou de sérum anti-peptide de procollagène (type III) et de procollagène (type III) ou respectivement de peptide de procollagène (type III) est séparé de l'antigène n'ayant pas réagi par addition d'un second anticorps dirigé contre l'antisérum hautement spécifique et séparation du complexe ainsi formé d'avec le résidu.

5. Procédé pour la fabrication de peptide de procollagène (type III) hautement purifié, approprié au procédé suivant l'une des revendications 1 à 4, caractérisé en ce que du tissu humain ou animal ou des humeurs pathologiques ou des extraits de collagène de ceux-ci sont dégradés avec de la collagénase et le procollagène ou peptide de procollagène ainsi formé est séparé et purifié par une combinaison de méthodes chromatographiques et/ou immuno-adsorption.

6. Procédé suivant la revendication 5, caractérisé en ce qu'il est utilisé en tant que tissu animal de la peau de veau foetal ou respectivement en tant que humeur du liquide ascitique humain.

7. Procédé pour la fabrication d'un sérum hautement spécifique anti-peptide de procollagène (type III), caractérisé en ce qu'un peptide de procollagène (type III) fabriqué suivant la revendication 5 ou 6 est utilisé pour l'immunisation d'animaux de laboratoire et le sérum de ces derniers est recueilli.